(19) Europäisches Patentamt / European Patent Office / Office européen des brevets

(11) **EP 3 848 452 A1**

(12) **EUROPEAN PATENT APPLICATION**
published in accordance with Art. 153(4) EPC

(43) Date of publication:
14.07.2021 Bulletin 2021/28

(21) Application number: 19858373.4

(22) Date of filing: 23.08.2019

(51) Int Cl.:
*C12N 1/00* (2006.01)   *B01D 15/00* (2006.01)
*B01J 20/02* (2006.01)   *B01J 20/18* (2006.01)
*B01J 20/26* (2006.01)

(86) International application number:
**PCT/JP2019/033126**

(87) International publication number:
**WO 2020/050069 (12.03.2020 Gazette 2020/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: 06.09.2018 JP 2018166843

(71) Applicants:
• **Nikkiso Co., Ltd.**
**Shibuya-ku, Tokyo 150-6022 (JP)**
• **Tohoku University**
**Sendai-shi, Miyagi 980-8577 (JP)**

(72) Inventors:
• **YOSHIOKA Toshiaki**
**Sendai-shi Miyagi 980-8577 (JP)**
• **KAMEDA Tomohito**
**Sendai-shi Miyagi 980-8577 (JP)**
• **KITAGAWA Fumihiko**
**Kanazawa-shi Ishikawa 920-0177 (JP)**
• **JIMBO Yoichi**
**Kanazawa-shi Ishikawa 920-0177 (JP)**
• **KONDO Masayuki**
**Kanazawa-shi Ishikawa 920-0177 (JP)**

(74) Representative: **Algemeen Octrooi- en Merkenbureau B.V.**
**P.O. Box 645**
**5600 AP Eindhoven (NL)**

(54) **AMMONIA ADSORBENT AND METHOD FOR REMOVING AMMONIA**

(57)     An ammonia adsorbent 4 includes at least one substance selected from the group consisting of L-zeolite, ferrierite, ZSM-5 zeolite, a strongly acidic cation exchange resin, and a Prussian blue complex.

FIG. 1A

EP 3 848 452 A1

**(Cont. next page)**

## FIG. 1B

## FIG. 1C

## FIG. 1D

**Description**

[TECHNICAL FIELD]

The present invention relates to an ammonia adsorbent and a method for removing ammonia.

[BACKGROUND ART]

In recent years, in fields such as pharmaceutical manufacturing and regenerative medicine, it has been required to artificially and efficiently mass-culture cells and microorganisms. Examples of cells for which mass culture is required include antibody-producing cells such as Chinese hamster ovary cells (CHO cells); and pluripotent stem cells such as embryonic stem cells (ES cells) and induced pluripotent stem cells (iPS cells). If these cells and the like can be stably cultured in large quantities for a long period of time, it becomes possible to efficiently produce biological substances such as monoclonal antibodies and differentiation-inducing tissues derived from pluripotent stem cells.

As a method for industrially mass-culturing cells and the like, suspension stirred culture with use of a culture tank such as a spinner flask may be feasible. The suspension stirred culture, however, tends to need large scale of equipment. It is, therefore, effective to increase the culture density of cells and the like in order to reduce costs. It is, however, known that increasing the culture density suppresses the proliferation of cells and the like. This is because the concentration of waste products (metabolites) in the culture solution (liquid medium) increases due to densification of the cells and the like, which reduces proliferative activity of the cells and the like. Ammonia is known as a typical waste product that affects cells and the like.

In order to stably grow cells and the like in a high-density condition, it is therefore desirable to remove ammonia accumulated in the culture solution. On the other hand, Patent Literature 1 for example discloses a cell culture apparatus in which a cell culture tank and a component adjusting solution tank are connected by a liquid feeding line provided with a culture solution component adjustment membrane that allows components to permeate depending on concentration difference. In this cell culture device, the waste products accumulated in the culture solution move to the component adjusting solution side, so that the concentration in the culture solution decreases. At the same time, the nutrients whose concentration has decreased during the culture are transferred from the component adjusting solution to the culture solution, and are replenished. The environment in the culture solution is thus maintained in a condition suitable for cell culture. The culture solution itself has been used as the component adjusting solution.

[Patent Literature]

[Patent Literature 1] WO2015/122528

[SUMMARY OF INVENTION]

[TECHNICAL PROBLEM]

The cell culture apparatus disclosed in Patent Literature 1 has removed waste products from the culture solution by use of the principle of dialysis. In order to attain sufficient removal of waste products, the capacity of the component adjusting solution tank has therefore been set to 10 times or more the capacity of the cell culture tank. There is therefore a problem that the required liquid amounts huge and becomes costly. In particular, in a case where the culture solution itself is used as the component adjusting solution, a large amount of expensive medium is consumed, which further increases the cost. In addition, in a case where the waste products are removed by using dialysis technology, there is also a problem that the structure of the culture apparatus becomes complicated.

Further, it is often desired to remove ammonia not only in cell culture solution but also in other solution systems. A novel ammonia removal technique by use of a technique other than the dialysis technique has therefore been strongly desired.

The present invention has been made in view of these circumstances, and one of the objects thereof is to provide a novel ammonia removal technique.

[SOLUTION TO PROBLEM]

In view of solving the above problems, one aspect of the present invention relates to an ammonia adsorbent. The ammonia adsorbent contains at least one substance selected from the group consisting of L-type zeolite, ferrierite, ZSM-5 type zeolite, strongly acidic cation exchange resin and Prussian blue type complex. According to this aspect, a novel ammonia removing technique can be provided.

In the above aspect, the ammonia adsorbent may come into contact with a solution containing ammonia, to adsorb ammonia in the solution. In addition, the solution may be a culture solution of cells or microorganisms, that contains glucose. Further, the amount of the ammonia adsorbent added to the solution may be more than 0.0005 g/mL and less than 0.2 g/mL. Further, the strongly acidic cation exchange resin may be an H-form strongly acidic cation exchange resin.

Another aspect of the present invention relates to a method for removing ammonia. The removing method includes bringing the ammonia adsorbent of any of the above aspects into contact with ammonia.

Note that also free combinations of these constituents, and also any of the constituents and expressions exchanged among the method, device and system, are valid as the aspects of the present invention.

[ADVANTAGEOUS EFFECTS OF INVENTION]

According to the present invention, a novel ammonia removing technique may be provided.

[BRIEF DESCRIPTION OF DRAWINGS]

Figs. 1(A) to 1(D) are schematic views for explaining a method for removing ammonia according to an embodiment.
Fig. 2 is a chart summarizing rates of ammonia adsorption of ammonia adsorbents in an aqueous ammonia solution.
Fig. 3 is a chart summarizing rates of glucose adsorption of the ammonia adsorbents in an aqueous solution containing ammonia and glucose.
Fig. 4 is a chart summarizing rates of ammonia adsorption and rates of glucose adsorption of the ammonia adsorbents in a cell culture solution.

[DESCRIPTION OF EMBODIMENTS]

The present invention will be explained below on the basis of preferred embodiments, referring to the attached drawings. The embodiments are merely illustrative, and are not restrictive about the invention. All features and combinations thereof described in the embodiments are not always necessarily essential to the present invention. All constituents, members and processes illustrated in the individual drawings will be given same reference numerals, so as to properly avoid redundant explanations. Reduced scales and shapes of the individual parts in the individual drawings are properly given for simplicity of explanation, and should not be interpreted restrictively unless otherwise specifically noted. Also note that ordinal terms "first", "second" and so on used in this patent specification and in claims do not represent any sequential order or importance, and are used only for discrimination of one structure from the other. Further, in each drawing, some of the members that are not important for explaining the embodiment will be omitted.

The present inventors have made extensive studies on the technique for removing ammonia, and have found an adsorbent capable of highly selectively adsorbing ammonia. Specifically, the ammonia adsorbent according to the present embodiment contains at least one substance selected from the group consisting of L-type zeolite, ferrierite, ZSM-5 type zeolite, strongly acidic cation exchange resin, and Prussian blue type complex. Ammonia can be adsorbed by bringing the ammonia adsorbent containing these substances into contact with ammonia.

In particular, the ammonia adsorbent of the present embodiment can be suitably used for adsorbing and removing ammonia in solution. In this case, the ammonia in solution can be adsorbed by bringing the ammonia adsorbent into contact with the ammonia-containing solution. Ammonia can be removed in this way. A plurality of types of ammonia adsorbents having different constituent substances may be mixed and used.

As the L-type zeolite, synthetic zeolite such as 500KOA (Tosoh Corporation) and HS-500 (FUJIFILM Wako Pure Chemical Corporation) can be used. The cation retained by these L-type zeolites is K (potassium). The retained ion is preferably K, but may alternatively be alkali metal, alkaline earth metal or lanthanoid such as Na, Li, Rb, Ce, Ba, Ca, Mg, Sr and La, or Al and Fe.

As the ferrierite, synthetic zeolites such as 720KOA (Tosoh) and HS-720 (FUJIFILM Wako Pure Chemical Corporation) can be used. The cation retained by these ferrierites is K (potassium). The retained ion is preferably K, but may be alkali metal, alkaline earth metal or lanthanoid, such as Na (770NAA), Li, Rb, Ce, Ba, Ca, Mg, Sr and La, or Al and Fe.

As the ZSM-5 type zeolite, synthetic zeolites such as 822HOA, 840HOA, 890HOA, and 891HOA (all by Tosoh Corporation) can be used. The cation retained by these ZSM-5 type zeolites is H (hydrogen).

These zeolites (L-type zeolite, ferrierite and ZSM-5 type zeolite) have a basic skeleton composed of silicon oxide, and a part of silicon in the basic skeleton is replaced with aluminum. Therefore, the entire crystal is negatively charged. Zeolites retain cations in the zeolite pores in order to maintain electrical neutrality. Cations are reversibly interchangeable. Zeolite, having K or H as the retained ion, can absorb ammonium (ammonium ion) in an improved manner.

As the strongly acidic cation exchange resin, PK216LH, PK216, SK112L (all by Mitsubishi Chemical Corporation), C150 (Purolite) and the like can be used. The strongly acidic cation exchange resin includes H-form strongly acidic cation exchange resin having $-SO_3H$ as an ion exchange group, and Na-form strongly acidic cation exchange resin having $-SO_3Na$ as an ion exchange group, bound to the basic skeleton having styrene and divinylbenzene polymerized therein. Aforementioned PL216LH belongs to the H-form, and PK216, SK112L and C150 belong to the Na-form. Ammonia is adsorbed by ion exchange between $H^+$ or $Na^+$ as an exchange group, and ammonium ion. The strongly acidic cation exchange resin is preferably the H-form strongly acidic cation exchange resin.

In addition, in a case where the solution is a glucose-containing culture solution of cells or microorganisms, the ammonia adsorbent of the present embodiment can adsorb ammonia to be removed, more selectively than glucose to be remained in the culture solution is adsorbed. Further, it is preferable to select an ammonia adsorbent having low toxicity to cells and microorganisms. The type of culture solution is not particularly limited.

The amount of the ammonia adsorbent added to the solution, in other words, the concentration of the ammonia adsorbent in the solution is preferably higher than 0.0005 g/mL, and more preferably 0.005 g/mL or higher. The amount of addition is preferably less than 0.2 g/mL, and more preferably 0.1 g/mL or less. With the amount of addition of the ammonia adsorbent set to more than 0.0005 g/mL, the rate of ammonia adsorption can be increased more reliably. Further, with the amount of addition set to 0.005 g/mL or more, a rate of ammonia adsorption of 10% or more is attainable in the culture solution. This makes it possible to more reliably reduce the amount of ammonia in the solution. The rate of ammonia adsorption is the ratio of the amount of adsorbed ammonia to the total amount of ammonia in the solution.

Further, with the amount of addition of the ammonia adsorbent set to less than 0.2 g/mL, the rate of glucose adsorption can be suppressed more reliably. Further, with the amount of addition set to 0.1 g/mL or less, a rate of glucose adsorption of 20% or less is attainable in the culture solution. As a result, the reduction in the amount of glucose caused by the ammonia adsorbent can be more reliably suppressed. Therefore, cells and the like can be cultured more efficiently. The rate of glucose adsorption is the ratio of the amount of adsorbed glucose to the total amount of glucose in the solution.

The cells and microorganisms cultured in the culture solution are not particularly limited. For example, cultured cells include pluripotent stem cells such as human iPS cells, human ES cells, and human Muse cells; somatic stem cells such as mesenchymal stem cells (MSC) and nephron progenitor cells; tissue cells such as human renal proximal tubule epithelial cells, human distal tubule epithelial cells, and human collecting duct epithelial cells; antibody-producing cell lines such as human fetal renal cells (HEK293 cells); antibody-producing cell lines derived from animals other than humans such as Chinese hamster ovary cells (CHO cells) and insect cells (SF9 cells). Since these cells are cells for which mass culture is particularly desired, they are more preferred targets to which the ammonia adsorbent of the present embodiment is applied.

Method for Removing Ammonia

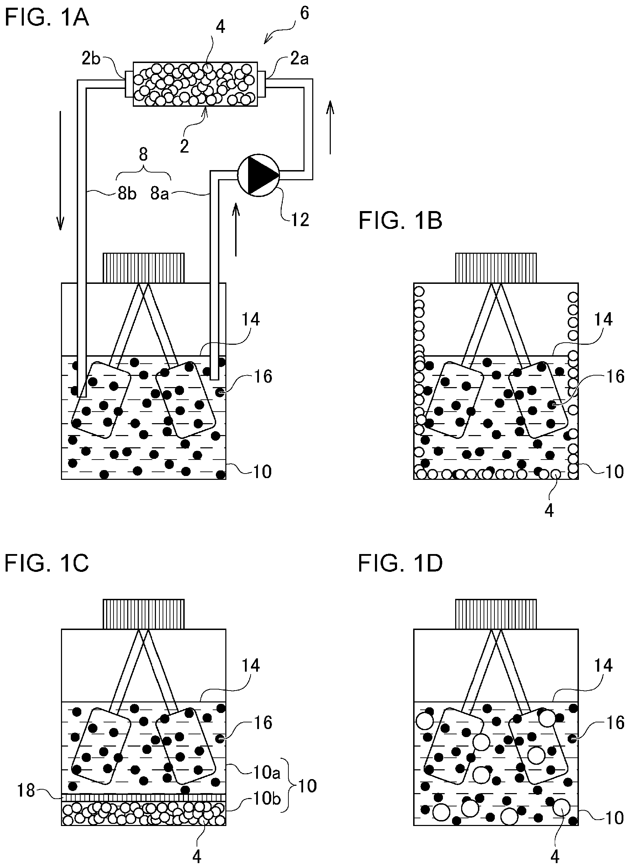

The method for removing ammonia according to the present embodiment includes bringing the above-mentioned ammonia adsorbent into contact with ammonia (ammonium ion). Preferably, the method involves bringing the ammonia adsorbent into contact with an ammonia-containing solution. The method for bringing the ammonia adsorbent into contact with ammonia is exemplified by the following aspects, although not particularly limited. Figs. 1(A) to 1(D) are schematic views for explaining a method for removing ammonia according to the embodiment. In the following, removal of ammonia from the culture solution will be described as an example. Also removal of ammonia from other solutions can be carried out in the same way.

In a first aspect as illustrated in Fig. 1(A), an adsorption module 6 having a container 2 such as a column packed with an ammonia adsorbent 4 is prepared. The container 2 has an inlet 2a and an outlet 2b through which the inside and the outside of the container 2 are communicated. The ammonia adsorbent 4 is, for example, in the form of particles. The adsorption module 6 is connected, through a circulation path 8, to a culture vessel 10 such as a spinner flask. The circulation path 8 includes an outward path 8a connecting the culture vessel 10 and the inlet 2a of the container 2, and a return path 8b connecting the outlet 2b of the container 2 and the culture vessel 10. A pump 12 is connected in the middle of the outward path 8a. The culture solution 14 and the cells 16 are housed in the culture vessel 10. The pump 12 may alternatively be arranged on the return path 8b.

When the pump 12 operates, the culture solution 14 is sucked from the culture vessel 10 and sent into the container 2 of the adsorption module 6 via the outward path 8a. The culture solution 14 fed into the container 2 is returned to the culture vessel 10 through the return path 8b. The culture solution 14 comes into contact with the ammonia adsorbent 4 packed in the container 2, in the process of circulating between the culture vessel 10 and the adsorption module 6. The ammonia in the culture solution 14 at this time is adsorbed by the ammonia adsorbent 4. Ammonia in the culture solution 14 is removed as a consequence. A filter (not illustrated) is provided at the end of the outward path 8a on the side connected to the culture vessel 10. The cells 16 are consequently suppressed from flowing towards the adsorption module 6. In the process of circulating the culture solution 14 between the culture vessel 10 and the adsorption module 6, medium components such as glucose and protein necessary for culturing the cells 16 may be replenished into the culture solution 14.

That is, in the first aspect, ammonia in the culture solution 14 is removed by using the culture apparatus provided with the adsorption module 6 that has the ammonia adsorbent 4, the culture vessel 10 that contains the cells or microorganisms as well as the culture solution 14, and the circulation path 8 that connects the adsorption module 6 and the culture vessel 10 so as to allow the culture solution 14 circulate.

As illustrated in Fig. 1(B), the ammonia adsorbent 4 of a second aspect is supported on the inner wall surface of the culture vessel 10. The culture vessel 10 houses the culture solution 14 and the cells 16. The culture solution 14 therefore comes into contact with the ammonia adsorbent 4 exposed on the inner wall surface of the culture vessel 10. This allows ammonia in the culture solution 14 to be adsorbed on the ammonia adsorbent 4. The culture vessel 10 is exemplified by spinner flask, petri dish, well plate, cell culture insert, and microsphere.

Method for supporting the ammonia adsorbent 4 on the inner wall surface of the culture vessel 10 is exemplified by a method of adhering the ammonia adsorbent 4 to the inner wall surface of the culture vessel 10, or, a method of molding the culture vessel 10, if it were made of resin, by using a resin preliminarily mixed with ammonia adsorbent 4. That is, in the second aspect, ammonia in the culture solution 14 is removed by using a culture apparatus that includes the culture vessel 10 and the ammonia adsorbent 4 supported on the inner wall surface of the culture vessel 10.

In a third aspect as illustrated in Fig. 1(C), the culture vessel 10 has a structure in which the inside of the vessel is divided into an upper part 10a and a lower part 10b by a diaphragm 18 such as a porous membrane. Such a culture vessel 10 is exemplified by a cell culture insert. The upper part 10a houses the culture solution 14 and the cells 16, and the lower part 10b houses the culture solution 14 and the ammonia adsorbent 4. The culture solution 14 can move back and forth between the upper part 10a and the lower part 10b through the diaphragm 18. In contrast, the cells 16 and the ammonia adsorbent 4 cannot pass through the diaphragm 18.

In such structure, the culture solution 14 comes into contact with the ammonia adsorbent 4 housed in the lower part 10b. This allows ammonia in the culture solution 14 to be adsorbed on the ammonia adsorbent 4. That is, in the third aspect, ammonia in the culture solution 14 is removed by using the culture apparatus provided with the culture vessel 10, the ammonia adsorbent 4, and the diaphragm 18 that divides the culture vessel 10 into a first space that houses the ammonia adsorbent 4 and a second space that houses the cells 16.

In a fourth aspect as illustrated in Fig. 1(D), the particulate ammonia adsorbent 4 is allowed to disperse, precipitate or suspend in the culture solution 14. This allows ammonia in the culture solution 14 to be adsorbed on the ammonia adsorbent 4. The ammonia adsorbent 4 preferably has a predetermined size or larger, for example 10 $\mu$m or larger, in view of preventing phagocytosis by the cells 16. That is, in the fourth aspect, the ammonia in the culture solution 14 is removed by using the culture apparatus provided with the culture vessel 10, and the ammonia adsorbent 4 added to the culture solution 14 in the culture vessel 10.

The ammonia adsorbent is preferably coated with a resin such as polyvinyl alcohol; a biological gel such as collagen, alginic acid, or gelatin, or the like. This suppresses outflow of fine particles that may affect cells and the like, out from the ammonia adsorbent into the culture solution. The ammonia adsorbent is alternatively formed by kneading ceramic binder, resin binder, biological gel or the like, with L-type zeolite or the like. This also makes it possible to suppress the outflow of fine particles. Examples of the ceramic binder include alumina binder and colloidal silica. Examples of the resin binder include polyvinyl alcohol, and carboxymethyl cellulose. Examples of the biological gel include collagen, alginic acid, and gelatin.

When detecting the ammonia concentration in the solution, it is preferable to use a medium component analyzer, although the method for detection is not particularly limited. The ammonia concentration can alternatively be detected by colorimetry by use of predetermined measurement reagent, enzyme electrode method utilizing the substrate specificity of enzyme, high performance liquid chromatography (HPLC), or the like.

As described above, the ammonia adsorbent according to the present embodiment contains at least one substance selected from the group consisting of L-type zeolite, ferrierite, ZSM-5 type zeolite, strongly acidic cation exchange resin and Prussian blue type complex. In addition, the method for removing ammonia according to the present embodiment includes bringing this ammonia adsorbent into contact with ammonia. This makes it possible to remove ammonia without using a huge amount of solution, unlike a known case where ammonia is removed by dialysis technique. The present embodiment can therefore provide a novel ammonia removing technique capable of removing ammonia at low cost. Further, since ammonia can be removed only by bringing the ammonia adsorbent into contact with the ammonia-containing solution, enabling the present embodiment to simplify structure of the culture apparatus.

Further, in a case where the solution is culture solution of cells and the like, the amount of consumption of the culture solution can be reduced as compared with known dialysis techniques. Since the culture solution is generally expensive, so that the cost can be further reduced. In addition, cells and the like can be mass-cultured at high density, by removing ammonia. Furthermore, in a case where the cells are pluripotent stem cells, the removal of ammonia not only enables high-density mass culture of the cells, but also can keep the cells in an undifferentiated stage, that is, the cells can remain pluripotent (can keep pluripotency). This therefore enables to obtain a large amount of cells suitable for producing biological substances and producing differentiation-inducing tissues. The cost required for drug manufacturing and regenerative medicine can therefore be reduced.

In addition, the ammonia adsorbent of the present embodiment can adsorb ammonia, more selectively than glucose, which is a useful component, is adsorbed. This therefore enables more efficient cell culture. Hence, the ammonia adsorbent of the present embodiment is particularly useful for removing ammonia in the glucose-containing culture solution. The ammonia adsorbent of the present embodiment may be used in combination with some adsorbent for other cell waste products.

The embodiments of the present invention have been described in detail above. The above-described embodiments merely illustrate specific examples in carrying out the present invention. Contents of the embodiments do not limit the technical scope of the present invention, instead allowing numerous design changes such as modification, addition, and deletion of constituents, without departing from the spirit of the present invention specified in the claims. Any new embodiment with design change will have effects derived both from an embodiment and modification to be combined. In the above-described embodiments, all contents possibly subject to such design change have been emphasized with a notation stating "... of the present embodiment" or "in the present embodiment". Also any content without such notation is, however, acceptable for the design change. Free combination of the above constituents is also valid as an aspect of the present invention.

[EXAMPLES]

Examples of the present invention will be explained below. Examples are merely illustrative, and by no means limit the present invention.

Performance Analyses of Adsorbents in Aqueous Ammonia Solution (Aqueous Solution)

Ammonium chloride (FUJIFILM Wako Pure Chemical Corporation) was added to pure water, to prepare an aqueous solution with an ammonia (ammonium ion) concentration of 10 mM. The pH of the aqueous solution was found to be 7.2. Then, 20 mL each of the aqueous solution was dispensed into a plurality of 50 mL Erlenmeyer flasks. Also 0.5 g each of various adsorbents was added to the aqueous solution in each flask. The concentration of the adsorbent was therefore 0.025 g/mL. The mixture was then shaken at 37°C and 150 rpm for 24 hours.

The adsorbents used are as follows.

Ceramic ($SiO_2$: Kanto Chemical Co., Inc.)
Activated carbon (FUJIFILM Wako Pure Chemical Corporation)
Metal oxide (MgO: Kanto Chemical Co., Inc.)
L-type zeolite (500KOA: Tosoh Corporation)
Ferrierite (720KOA: Tosoh Corporation)
Mordenite (642NAA: Tosoh Corporation)
Y-type zeolite (320NAA: Tosoh Corporation)
ZSM-5 type zeolite (822HOA: Tosoh Corporation)
Porous H-form strongly acidic cation exchange resin (PK216LH: Mitsubishi Chemical Corporation)
Porous Na-form strongly acidic cation exchange resin (PK216: Mitsubishi Chemical Corporation)
Gel-type, Na-form strongly acidic cation exchange resin (SK112L: Mitsubishi Chemical Corporation)
Prussian blue type complex (Prussian blue: Kanto Chemical Co., Inc.)

The cation retained by mordenite and Y-type zeolite is Na. The gel-type strongly acidic cation exchange resin is composed of a three-dimensional structure of a polymer of styrene and divinylbenzene. The porous type one is composed of a porous structure in which pores are physically provided in the three-dimensional structure of the gel-type one.

After 24 hours, the aqueous solution and the adsorbent were separated by a 0.1 $\mu$m filter. Ammonia concentration in the aqueous solution was then measured using an HPLC apparatus (JASCO Corporation). In addition, the adsorption rate of ammonia of each adsorbent was calculated from the equation below.

$$\text{Adsorption rate (\%) = \{[Concentration before adsorption} - \text{Concentration after adsorption]/Concentration before adsorption\}} \times 100$$

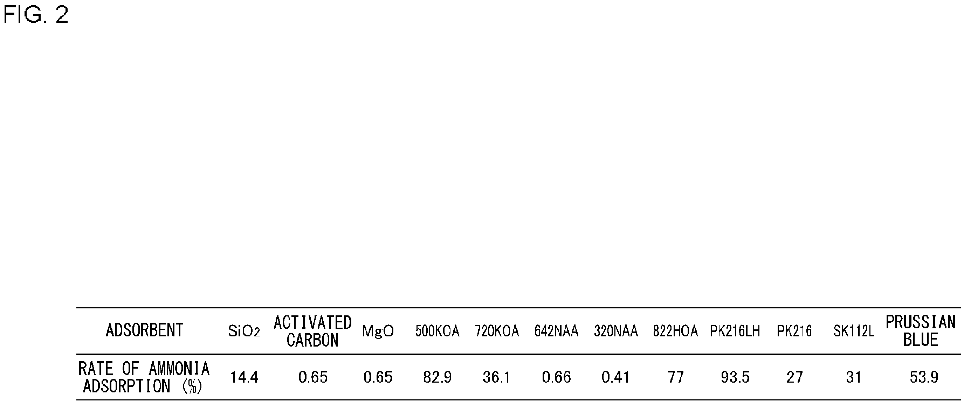

Results are summarized in Fig. 2. Fig. 2 is a chart summarizing the rates of ammonia adsorption of the ammonia adsorbents in the aqueous ammonia solution. As summarized in Fig. 2, at an adsorbent concentration of 0.025 g/mL, each of L-type zeolite (500KOA), ferrierite (720KOA), ZSM-5 type zeolite (822HOA), H-form strongly acidic cation exchange resin (PK216LH), Na-form strongly acidic cation exchange resins (PK216 and SK112L) and the Prussian blue type complex (Prussian blue) demonstrated a rate of ammonia adsorption of 20% or larger. On the other hand, each of ceramic ($SiO_2$), activated carbon, metal oxide (MgO), mordenite (642NAA) and Y-type zeolite (320NAA) was found to demonstrate a rate of ammonia adsorption of 15% or smaller.

In addition, even the Na-form strongly acidic cation exchange resin (PK216), which demonstrated the lowest rate of ammonia adsorption in an adsorbent group with higher rates of ammonia adsorption, was found to demonstrate nearly twice the rate of ammonia adsorption of ceramic ($SiO_2$), which demonstrated the highest rate of ammonia adsorption in an adsorbent group with lower rates of ammonia adsorption.

It was consequently confirmed that L-type zeolite, ferrierite, ZSM-5 type zeolite, strongly acidic cation exchange resins and Prussian blue type complex demonstrated excellent ammonia adsorption ability. When compared among the strongly acidic cation exchange resins, the H-form strongly acidic cation exchange resin (PK216LH) was confirmed to have higher ability to adsorb ammonia than the Na-form strongly acidic cation exchange resins (PK216 and SK112L).

Performance Analyses of Adsorbents in Aqueous Solution Containing Ammonia and Glucose (Aqueous Solution)

Ammonium chloride (Kanto Chemical Co., Inc.) and glucose (Kanto Chemical Co., Inc.) were added to pure water to prepare an aqueous solution having a glucose concentration of 1000 ppm and an ammonia (ammonium ion) concentration of 10 mM. The pH of the aqueous solution was found to be 7.2. Then, 20 mL each of the aqueous solution was dispensed into a plurality of 50 mL Erlenmeyer flasks.

Each of L-type zeolite (500KOA), ferrierite (720KOA), ZSM-5 type zeolite (822HOA), H-form strongly acidic cation exchange resin (PK216LH), Na-form strongly acidic cation exchange resin (PK216 and SK112L) and Prussian blue type complex (Prussian Blue), having demonstrated good ammonia adsorption ability in the aforementioned adsorption test, was then added to the aqueous solution in each flask. In addition, each of ceramic ($SiO_2$), activated carbon and metal oxide (MgO) was also added to the aqueous solution in each flask, for reference. Amount of addition of each adsorbent was 0.5 g. The concentration of the adsorbent was therefore 0.025 g/mL. The mixture was then shaken at 37°C and 150 rpm for 24 hours.

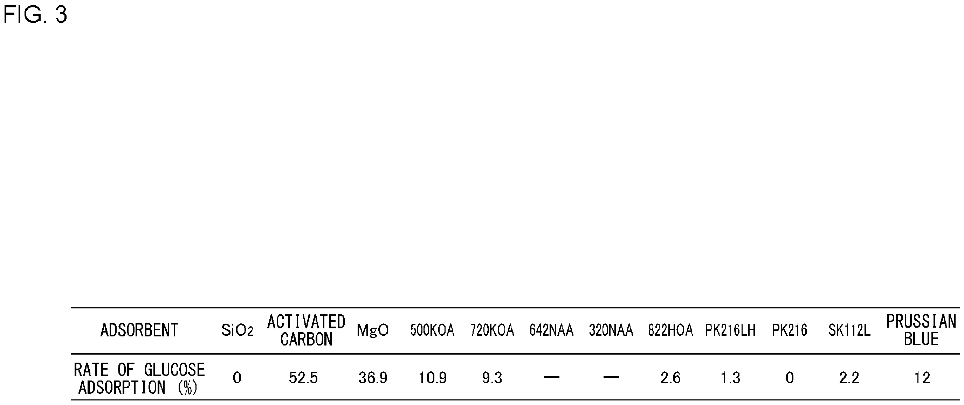

After 24 hours, the aqueous solution and the adsorbent were separated by a 0.1 μm filter. Glucose concentration in the aqueous solution was then measured by using an HPLC apparatus (JASCO Corporation), and the rate of glucose adsorption of each adsorbent was calculated from the equation above. Results are summarized in Fig. 3. Fig. 3 is a chart summarizing rates of glucose adsorption of the ammonia adsorbents in an aqueous solution containing ammonia and glucose.

As summarized in Fig. 3, the rate of glucose adsorption of each of L-type zeolite, ferrierite, ZSM-5 type zeolite, strongly acidic cation exchange resins and Prussian blue type complex was 20% or smaller. On the other hand, each of activated carbon and metal oxide (MgO) was found to demonstrate a rate of glucose adsorption of 30% or larger. It was consequently confirmed that L-type zeolite, ferrierite, ZSM-5 type zeolite, strongly acidic cation exchange resins and Prussian blue type complex can adsorb ammonia, more selectively than glucose is adsorbed. Ceramic ($SiO_2$) demonstrated a rate of glucose adsorption of 0%, but only a low rate of ammonia adsorption as described above, thus confirmed to be inferior to the ammonia adsorbent of the present embodiment in terms of performance.

Performance Analyses of Adsorbents in Cell Culture Solution

Ammonium chloride (FUJIFILM Wako Pure Chemical Corporation) was added to a pluripotent stem cell medium (StemFit AK02N: Ajinomoto Co., Inc.) to prepare a medium having a glucose concentration of 250 mg/dL and an ammonia (ammonium ion) concentration of 10 mM. Twenty milliliters each of the medium was dispensed into each of a plurality of 50 mL tubes (Thermo Fisher Scientific Inc.). Then, 0.5 g (0.025 g/mL) each of various adsorbents was added to the medium in each tube. Adsorbents used include L-type zeolite (500KOA), ferrierite (720KOA), ZSM-5 type zeolite (822HOA), porous H-form strongly acidic cation exchange resin (PK216LH) and Prussian blue type complex (Prussian Blue). The mixture was then shaken at 37°C and 60 rpm for 24 hours.

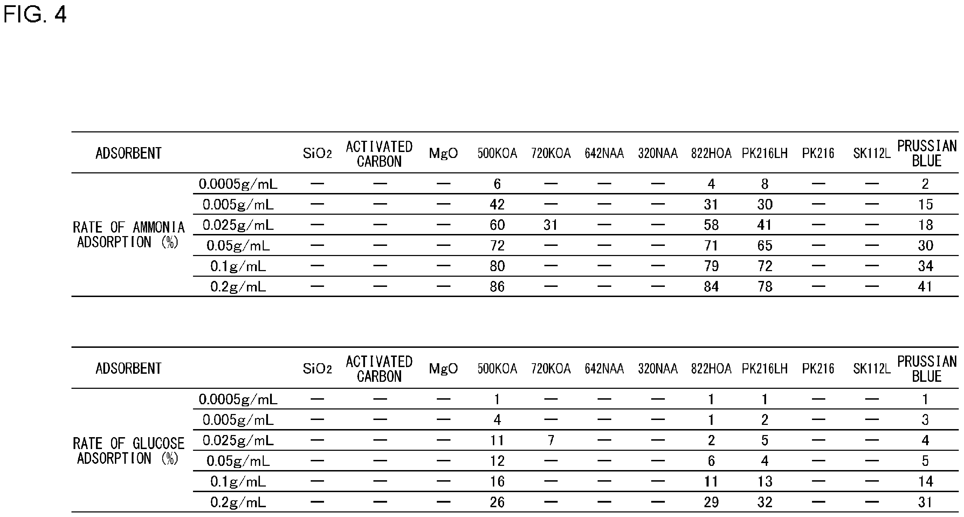

After 24 hours, the culture solution and the adsorbent were separated by a 0.22 μm filter. The ammonia concentration in the cell culture solution was then measured by using Ammonia Assay Kit (Sigma-Aldrich), and the glucose concentration in the cell culture solution was measured by using a blood gas analyzer (ABL800 FLEX: Radiometer Medical ApS). The adsorption rate of ammonia and the adsorption rate of glucose of each adsorbent were then calculated from the equation above. Results are summarized in Fig. 4.

The adsorbents other than ferrierite (720KOA) were also subjected to the aforementioned adsorption test, at different amounts of addition to the medium, and the rate of ammonia adsorption and the rate of glucose adsorption were calculated. The amount of addition (concentration) was varied among 0.01 g (0.0005 g/mL), 0.1 g (0.005 g/mL), 0.5 g (0.025 g/mL), 1.0 g (0.05 g/mL), 2.0 g (0.1 g/mL) and 4.0 g (0.2 g/mL). Results are summarized in Fig. 4.

Fig. 4 is a chart summarizing the rate of ammonia adsorption and the rate of glucose adsorption of the ammonia adsorbents in the cell culture solution. As summarized in Fig. 4, L-type zeolite, ferrierite, ZSM-5 type zeolite, strongly acidic cation exchange resin and Prussian blue type complex were confirmed to adsorb ammonia also in the cell culture solution at an adsorbent concentration of 0.025 g/mL, although becoming slightly lower than in the aqueous solution. The rates of glucose adsorption were found to be 20% or smaller. From this, these adsorbents were confirmed to adsorb ammonia, more selectively than glucose is adsorbed, also in the cell culture solution.

It was also confirmed that ammonia can be adsorbed at any adsorbent concentration. Further improved rate of ammonia adsorption was confirmed to be attainable particularly at an adsorbent concentration of higher than 0.0005 g/mL, and further 0.005 g/mL or higher. The rate of ammonia adsorption was also found to be higher at all adsorbent concentrations, than the rate of glucose adsorption. In addition, these adsorbents were found to have a rate of glucose adsorption of 32% at the maximum. It was thus confirmed that these adsorbents are suitable for removing ammonia in the medium at any adsorbent concentration.

Note that the rate of ammonia adsorption increased as the concentration of the adsorbent increased, but concurrently the rate of glucose adsorption also increased. It was however confirmed that the rate of glucose adsorption can be reduced more satisfactorily, if the adsorbent concentration is adjusted to lower than 0.2 g/mL, and preferably to 0.1 g/mL or lower.

[INDUSTRIAL APPLICABILITY]

The present invention is applicable to an ammonia adsorbent and a method for removing ammonia.

[REFERENCE SIGNS LIST]

2 container, 4 ammonia adsorbent, 6 adsorption module, 8 circulation path, 10 culture vessel, 12 pump, 14 culture solution, 16 cell, 18 diaphragm

**Claims**

1. An ammonia adsorbent comprising at least one substance selected from the group consisting of L-type zeolite, ferrierite, ZSM-5 type zeolite, strongly acidic cation exchange resin and Prussian blue type complex.

2. The ammonia adsorbent according to claim 1, allowed for contact with an ammonia-containing solution to adsorb ammonia in the solution.

3. The ammonia adsorbent according to claim 2, wherein the solution is a culture solution of cells or microorganisms, that contains glucose.

4. The ammonia adsorbent according to claim 2 or 3, wherein the amount of addition thereof to the solution is more than 0.0005 g/mL and less than 0.200 g/mL.

5. The ammonia adsorbent according to any one of claims 1 to 4, wherein the strongly acidic cation exchange resin is an H-form strongly acidic cation exchange resin.

6. A method for removing ammonia, the method comprising bringing the ammonia adsorbent described in any one of claims 1 to 5 into contact with ammonia.

FIG. 1A

FIG. 1B

FIG. 1C

FIG. 1D

FIG. 2

| ADSORBENT | SiO$_2$ | ACTIVATED CARBON | MgO | 500KOA | 720KOA | 642NAA | 320NAA | 822HOA | PK216LH | PK216 | SK112L | PRUSSIAN BLUE |
|---|---|---|---|---|---|---|---|---|---|---|---|---|
| RATE OF AMMONIA ADSORPTION (%) | 14.4 | 0.65 | 0.65 | 82.9 | 36.1 | 0.66 | 0.41 | 77 | 93.5 | 27 | 31 | 53.9 |

FIG. 3

EP 3 848 452 A1

| ADSORBENT | SiO$_2$ | ACTIVATED CARBON | MgO | 500KOA | 720KOA | 642NAA | 320NAA | 822HOA | PK216LH | PK216 | SK112L | PRUSSIAN BLUE |
|---|---|---|---|---|---|---|---|---|---|---|---|---|
| RATE OF GLUCOSE ADSORPTION (%) | 0 | 52.5 | 36.9 | 10.9 | 9.3 | — | — | 2.6 | 1.3 | 0 | 2.2 | 12 |

# FIG. 4

| ADSORBENT | | SiO₂ | ACTIVATED CARBON | MgO | 500KOA | 720KOA | 642NAA | 320NAA | 822HOA | PK216LH | PK216 | SK112L | PRUSSIAN BLUE |
|---|---|---|---|---|---|---|---|---|---|---|---|---|---|
| RATE OF AMMONIA ADSORPTION (%) | 0.0005g/mL | — | — | — | 6 | — | — | — | 4 | 8 | — | — | 2 |
| | 0.005g/mL | — | — | — | 42 | — | — | — | 31 | 30 | — | — | 15 |
| | 0.025g/mL | — | — | — | 60 | 31 | — | — | 58 | 41 | — | — | 18 |
| | 0.05g/mL | — | — | — | 72 | — | — | — | 71 | 65 | — | — | 30 |
| | 0.1g/mL | — | — | — | 80 | — | — | — | 79 | 72 | — | — | 34 |
| | 0.2g/mL | — | — | — | 86 | — | — | — | 84 | 78 | — | — | 41 |

| ADSORBENT | | SiO₂ | ACTIVATED CARBON | MgO | 500KOA | 720KOA | 642NAA | 320NAA | 822HOA | PK216LH | PK216 | SK112L | PRUSSIAN BLUE |
|---|---|---|---|---|---|---|---|---|---|---|---|---|---|
| RATE OF GLUCOSE ADSORPTION (%) | 0.0005g/mL | — | — | — | 1 | — | — | — | 1 | 1 | — | — | 1 |
| | 0.005g/mL | — | — | — | 4 | — | — | — | 1 | 2 | — | — | 3 |
| | 0.025g/mL | — | — | — | 11 | 7 | — | — | 2 | 5 | — | — | 4 |
| | 0.05g/mL | — | — | — | 12 | — | — | — | 6 | 4 | — | — | 5 |
| | 0.1g/mL | — | — | — | 16 | — | — | — | 11 | 13 | — | — | 14 |
| | 0.2g/mL | — | — | — | 26 | — | — | — | 29 | 32 | — | — | 31 |

<table>
<tr><td colspan="2" align="center"><b>INTERNATIONAL SEARCH REPORT</b></td><td colspan="2">International application No.<br>PCT/JP2019/033126</td></tr>
</table>

**A. CLASSIFICATION OF SUBJECT MATTER**

Int.Cl. C12N1/00(2006.01)i, B01D15/00(2006.01)i, B01J20/02(2006.01)i, B01J20/18(2006.01)i, B01J20/26(2006.01)i

According to International Patent Classification (IPC) or to both national classification and IPC

**B. FIELDS SEARCHED**

Minimum documentation searched (classification system followed by classification symbols)

Int.Cl. C12N1/00, B01D15/00, B01J20/02, B01J20/18, B01J20/26

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

CAplus/MEDLINE/EMBASE/BIOSIS (STN), JSTPlus/JMEDPlus/JST7580 (JDreamIII), PubMed

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2017-104778 A (HIROSHIMA UNIVERSITY et al.) 15 June 2017, claims 1, 3 & US 2018/0323457 A1, claims 1, 3 & WO 2017/099149 A1 & EP 3388145 A1 | 1,5,6<br>1-6 |

☒ Further documents are listed in the continuation of Box C. ☐ See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 22 November 2019 (22.11.2019) | 03 December 2019 (03.12.209) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

| International application No. |
|---|
| PCT/JP2019/033126 |

C (Continuation).  DOCUMENTS CONSIDERED TO BE RELEVANT

| Category* | Citation of document, with indication, where appropriate, of the relevant passages | Relevant to claim No. |
|---|---|---|
| X<br>Y | WO 2011/034039 A1 (MEDICAL CARE PROTEOMICS BIOTECHNOLOGY CO., LTD.) 24 March 2011, claims 1-3, paragraph [0020] & CN 102695516 A & KR 10-2012-0051776 A | 1,2,5,6<br>1-6 |
| X<br>Y | WO 2015/186819 A1 (NATIONAL INSTITUTE OF ADVANCED INDUSTRIAL SCIENCE AND TECHNOLOGY) 20 December 2015, claims 1, 3, examples 1, 2 & US 2017/0096348 A1, claim 10, examples 1, 2 | 1,2,4-6<br>1-6 |
| Y | NAYVE, FR Jr. et al., "Selective removal of ammonia from animal cell culture broth.", Cytotechnology, 1991, vol. 6, no. 2, pp. 121-130, abstract, page 126, right column, paragraph [0002] | 1-6 |
| A | JP 2009-072129 A (HITACHI PLANT TECHNOLOGIES, LTD.) 09 April 2009, entire text, all drawings (Family: none) | |
| A | 窪田好浩, et al., ゼオライト開発の現状, J. Vac. Soc. Jpn., 2006, vol. 49, no. 4, pp. 205-212 entire text, all drawings, (KUBOTA, Yoshihiro et al., "Recent Developments in Zeolite Science and Technology") | 1-6 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015122528 A